# EUROPEAN PATENT APPLICATION

(11) **EP 2 036 829 A1**
(43) Date of publication of application: **18.03.2009**
(21) Application number: 08156835.4
(22) Date of filing: 23.05.2008
(51) Int. Cl.: B65D 51/00, A61J 1/14

(54) **A container top and a method of protecting a container top septum**

(30) Priority: 12.09.2007 US 854203
(71) Applicant: Integrated Liner Technologies, Inc., Albany, NY 12202 (US)
(72) Inventor: Petrosino, Paul, Schoharie, NY NY 12157 (US); Crouch, James, E. Greenbush, NY NY 12061 (US); Frake, Kevin, Bristol, PA PA 19007 (US); Faulkner, Michael, Gardner, MA MA 01440 (US)
(74) Representative: Ansala, Jyrki Matti

(57) **Abstract**

A container top having an integral cover (33), for example, an integral dust cover, is provided. The container top includes a cap (32) having a cylindrical skirt (34) with a top (36), a bottom (38), a first outside diameter (46) at the top (36) of the skirt (34), a second outside diameter (48) at the bottom (38) of the skirt (34), and an inside surface (50) adapted to engage a container; and a ring (40) mounted to the top (36) of the cylindrical skirt (34), the ring (40) having an opening (42); and a cover (33) removably mounted to the cap (32) and occluding the opening (42) in the ring (40). The container top may include a septum (35) positioned in the opening (42), for example, a septum (35) that can be pierced with a syringe. The container cap (32) may be used in a wide range of industries, for example, the medical, pharmaceutical, the general scientific community, or in any industry where liquid or gaseous samples are handled.

## Description

### TECHNICAL FIELD

The present invention relates to a perforated cap having an integral cover. More particularly, the present invention relates to a perforated cap having a flexible septum and a removable protective cover protecting the flexible septum during handling and storage.

### BACKGROUND OF THE INVENTION

Container caps having apertures in their tops and flexible sealing elements mounted across the apertures are common in the medical, pharmaceutical, and general scientific community for storing samples, sera, and other liquid or gaseous contents. The flexible sealing element, or septum, typically provides a fluid-tight seal between the apertured cap and the container, for example, a vial, to which the cap is mounted while permitting access to the inside of the container by piercing the septum with, for example, a syringe, to introduce a fluid or withdraw a fluid.

Due to the nature of the contents of these vials or containers in the medical and pharmaceutical fields, among others, it is typically imperative that such caps and septa be protected from pollutants and contaminants during use, transfer, storage, and other handling. For example, the Environmental Protection Agency (EPA) has established standards under 40 C.F.R. §136 entitled "Guidelines for Establishing Test Procedures for the Analysis of Pollutants" and 40 C.F.R. § 141 entitled "National Interim Primary Drinking Water Regulations: Control of Trihalomethanes in Drinking Water," among others, which describe acceptable testing procedures for the handling of samples during water analysis. These EPA standards specify a vial/cap/septum construction as well as detailed cleaning and sampling procedure that are designed to ensure that the sample is not contaminated or lost prior to analysis. These EPA regulations specify an open top cap and a septum comprised of silicone rubber laminated with polytetrafluoroethylene (PTFE), for example, Dupont's Teflon PTFE, where the face of the PTFE lamination is exposed to the sample in the vial.

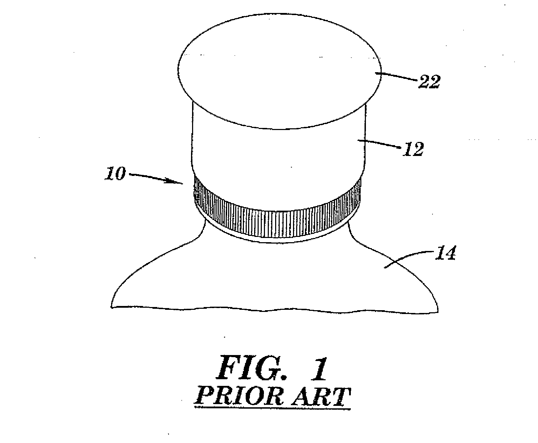

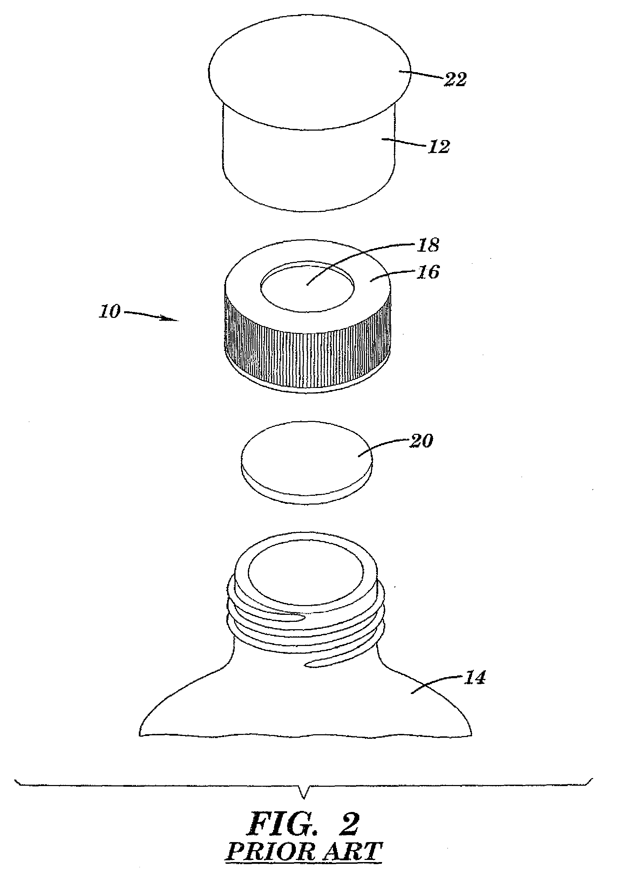

Among other things, these EPA procedures specify that the exposed elastomeric septum in the cap be protected from contamination during shipping, storage, and other handling. According to conventional practice, the caps and septa are typically protected by a removal cap or "dust cover," for example, as shown and described with respect to FIGURES 1 and 2 below. These removable, typically, plastic, caps are typically manually mounted to the cap and septum while the cap and septum are mounted on the vial prior to transport or storage. Subsequently, the caps are removed to permit access to the cap and septum.

However, these removable dust covers and caps to which the dust covers are mounted are typically provided by separate suppliers and, prior to assembly by the end user, must each be cleaned. In accordance with conventional practice, the end user typically washes both the dust cover and the cap prior to assembly. This washing and handling by the end user is not only labor intensive, but also introduces the potential of introducing undesirable contaminants, including moisture, between the dust cover and the cap during the washing and handling process. Therefore, there exists a need for a cap having an integrated dust cover that overcomes the disadvantages of the prior art. Aspects of the present invention address this need while having other advantages compared to the prior art.

### SUMMARY OF THE INVENTION

Aspects of the present invention eliminate the need of a separate dust cover and its consequent handling and washing. One aspect of the invention is a container top comprising a cap with a cylindrical skirt having a top, a bottom, a first outside diameter at the top of the skirt, a second outside diameter at the bottom of the skirt, and an inside surface adapted to engage a container; and a ring mounted to the top of the cylindrical skirt, the ring having an opening; and a cover removably mounted to the cap and occluding the opening in the ring, the cover having substantially no structure extending beyond the first outside diameter of the skirt. In one aspect, the container top further includes a septum positioned in the cap to occlude the opening in the ring. In another aspect, the ring includes a first annular retaining structure, such as, an annular recess or an annular projection, and the cover includes a complementary second annular retaining structure adapted to engage the first annular retaining structure to retain the cover on the cap. In another aspect, the first annular retaining structure and the second annular retaining structure comprise the only means of engagement between the cap and the cover.

Another aspect of the invention is a method of protecting a cap septum including providing a cap comprising a cylindrical skirt having top, a bottom, a first outside diameter at the top of the skirt, and a second outside diameter at the bottom of the skirt; and a ring mounted to the top of the cylindrical skirt, the ring having an opening; inserting a septum into the cap wherein the septum occludes the opening; mounting the cap with the septum on a container wherein at least some of the septum is exposed through the opening in the ring; and mounting a removable cover on the cap to protect the septum while providing no structure extending beyond the first outside diameter of the skirt. In one aspect, the cap further comprises a first annular retaining structure and the cover comprises a second annular retaining structure, and wherein mounting the removable cover on the cap comprise engaging the first annular retaining structure with the second annular retaining structure.

A further aspect of the invention is a container top having a cap including a cylindrical skirt having a top, a bottom, a first outside diameter at the top of the skirt, a second outside diameter at the bottom of the skirt, and an inside surface adapted to engage a container; and a ring mounted to the top of the cylindrical skirt, the ring having an opening and a first annular retaining structure; and a cover removably mounted to the cap and occluding the opening in the ring, the cover having a second annular retaining structure adapted to engage the first annular retaining structure of the ring to retain the cover on the cap. In one aspect, the container top further comprises a septum positioned in the cap to occlude the opening in the ring. In another aspect, the first annular retaining structure comprises one of an annular recess and an annular projection. In one aspect, the cover comprises substantially no structure extending beyond the first outside diameter of the skirt.

These and other aspects, features, and advantages of this invention will become apparent from the following detailed description of the various aspects of the invention taken in conjunction with the accompanying drawings

### BRIEF DESCRIPTION OF THE DRAWINGS

The subject matter, which is regarded as the invention, is particularly pointed out and distinctly claimed in the claims at the conclusion of the specification. The foregoing and other objects, features, and advantages of the invention will be readily understood from the following detailed description of aspects of the invention taken in conjunction with the accompanying drawings in which:

FIGURE 1 is a perspective view of a typical prior art cap assembly with a dust cover mounted to a bottle.

FIGURE 2 is an exploded perspective view of the prior art cap assembly with a dust cover mounted to a bottle shown in FIGURE 1.

FIGURE 3 is perspective view of an integral cap and dust cover assembly according to one aspect of the invention.

FIGURE 4 is an exploded perspective view of the integral cap and dust cover assembly shown in FIGURE 3.

FIGURE 5 is a cross sectional view of the dust cover shown in FIGURE 4.

FIGURE 6 is a cross sectional view of the cap shown in FIGURE 4.

FIGURE 7 is a cross sectional view of the assembled dust cover and cap shown in FIGURES 5 and 6.

FIGURE 8 is a perspective view of another cap and cover according to another aspect of the invention.

FIGURE 9 is a perspective view of the cap and cover shown in FIGURE 8 with the cover opened.

FIGURE 10 is a cross sectional view of the cap and cover shown in FIGURE 8.

### DETAILED DESCRIPTION

FIGURE 1 is a perspective view of a typical prior art cap assembly 10 having a dust cover 12 mounted to a bottle or vial 14 (only the top portion of which is shown).
FIGURE 2 is an exploded perspective view of the prior art cap assembly 10 having dust cover 12 mounted to bottle 14 shown in FIGURE 1. As shown, prior art cap assembly 10 may typically include a cap 16 having a hole 18 in its top and a septum 20 mounted in cap 16 to obstruct hole 18, for example, mounted by means of the methods disclosed in commonly-assigned U.S. patents 5,647,939 and 6,234,335 -- the disclosures of which is included by reference herein. Septum 20 may typically be an inert elastomeric material as disclosed in the above referenced patents, for example, a PTFE-laminated silicone rubber, or its equivalent. As is known in the art, septum 20 provides a piercible medium through which, for example, a syringe may be passed to access the contents of bottle 14 while minimizing or preventing leakage of the contents through the hole created by the syringe. According to prior art practice, dust cover 12 is provided for cap assembly 10 to minimize or prevent the contamination of cap 16 and septum 20 during handling, transport, and storage. According to the prior art, dust cover 12 typically comprises a molded plastic cover sized to fit snugly about cap 16 and protect hole 18 and septum 20 from contamination. Typically, dust cover 12 is manually mounted to cap 16 and manually removed from cap 16 by a technician, for example, by prying dust cover 12 from cap 16 by grasping annular lip 22 on dust cover 12.

However, the presence, handling, and manipulation of dust cover 12 can be problematic. For example, dust cover 12 may be made from a material that can contaminate cap assembly 10, specifically contaminate septum 20. For example, when dust cover 12 is fabricated from an inadequate thermoplastic, the dust cover 12 can react or otherwise contaminate the material from which septum 20 is made, for example, a silicone rubber. Also, the presence of dust cover 12 upon arrival at a facility requires that dust cover 12 be removed from cap assembly 10, discarded, or washed with cap assembly 12 prior to use or re-use. For these and other disadvantages of the prior art shown in FIGURES 1 and 2, the present invention in its various aspects was conceived and developed.

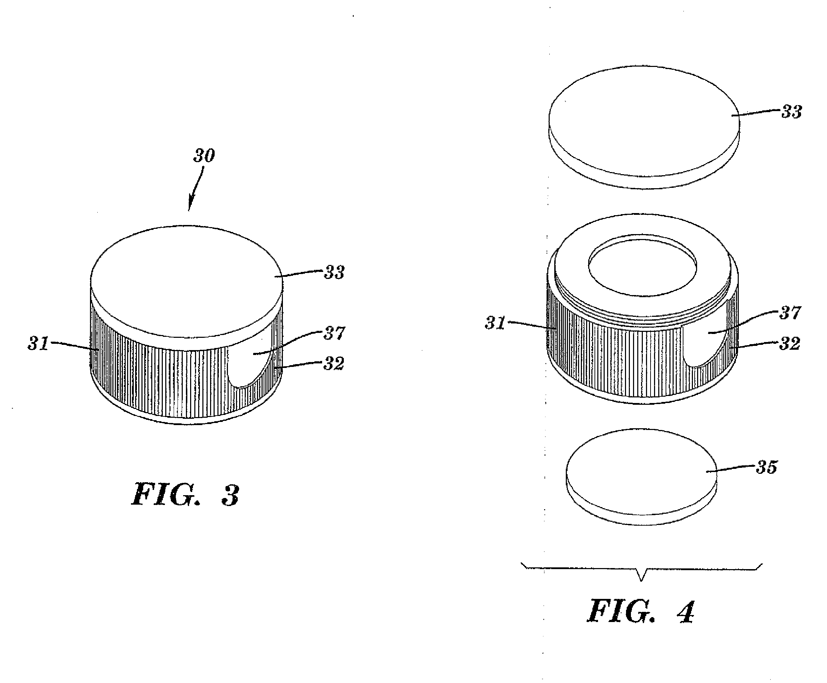

FIGURE 3 is perspective view of an integral cap and dust cover assembly, or container top, 30 according to one aspect of the invention. FIGURE 4 is an exploded perspective view of the integral cap and dust cover assembly 30 shown in FIGURE 3. According to aspects of the present invention, container top 30 includes a cap 32 and a cover, or dust cover, 33. In one aspect of the invention, cap 32 may include a septum 35, for example, an elastomeric septum similar to septum 20 discussed above.

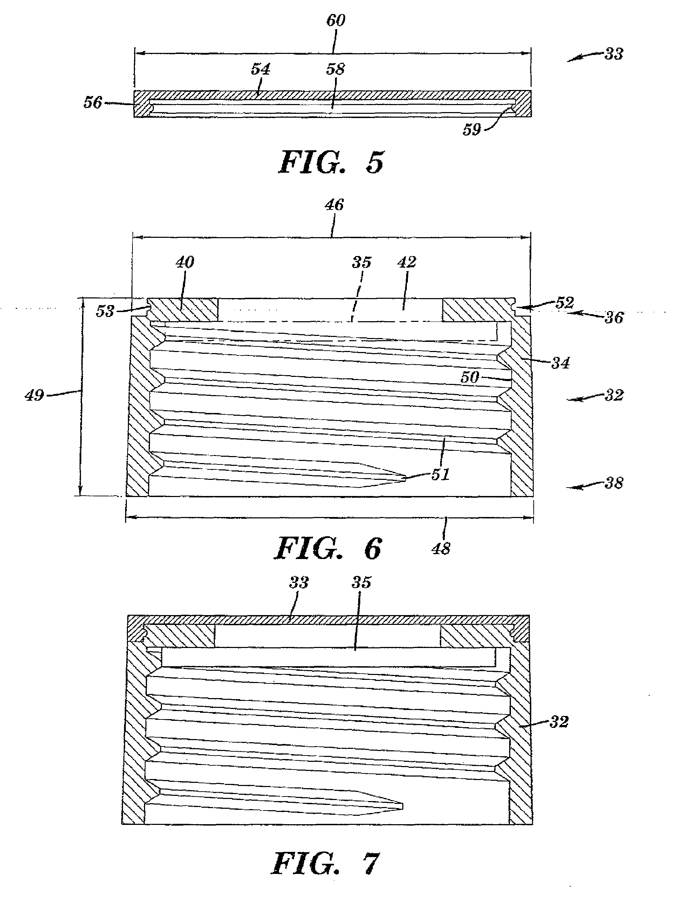

FIGURE 5 is a cross sectional view of cover 33 shown in FIGURE 4. FIGURE 6 is a cross sectional view of cap 32 shown in FIGURE 4. FIGURE 7 is a cross sectional view of the assembled cover 33 and cap 32 shown in FIGURES 5 and 6. As shown in FIGURES 3 and 4 container top 30 may include an outside surface 31 that is typically textured or knurled to facilitate handling of top 30, for example, when threading or unthreading container top 30 from bottle 14. As also shown, container top 30 may include a recess 37 in surface 31 to facilitate access to cap 32, for example, for removing cap 32.

As shown most clearly in FIGURE 6, cap 32 includes a cylindrical skirt 34 having a top or first end 36 and a bottom or second end 38, and a ring 40 mounted to the top 36. Ring 40 includes an opening 42 which exposes septum 35 (shown in phantom in FIGURE 6) mounted within cap 32, for example, by means of the methods disclosed in U.S. patents 5,647,939 and 6,234,335. Septum 35 may include markings to facilitate positioning a syringe for insertion through septum 35, for example, one or more circles or "cross hairs." The markings may be recessed into the surface of septum 35 or protrude above the surface of septum 35.

According to an aspect of the invention, skirt 34 includes a first outside diameter 46 at the top 36 of skirt 34, a second outside diameter 48 at the bottom 38 of skirt 34, a height 49, and an inside surface 50 adapted to engage a container (not shown), for example, a bottle similar to bottle 14 shown in FIGURES 1 and 2. The inside diameter 50 may include one or more helical threads 51 adapted to engage a threaded bottleneck. In one aspect, first outside diameter 46 may be substantially equal to second outside diameter 48; however, first outside diameter 46 may be smaller than second outsider diameter 48, for instance, to facilitate removal of cap 32 from a mold when cap 32 is fabricated by molding. Outside diameter 46 may also be greater than outside diameter 48.

As also shown in FIGURE 6, cap 32 may include an annular retaining structure 52, for example, a first annular retaining structure, adapted to engage an annular retaining structure, for example, a second annular retaining structure, in cover 33, for example, annular retaining structure 58 discussed below. In one aspect, the annular retaining structure 52 of cap 32 may be associated with ring 40, for example, annular retaining structure 52 may be mounted on the inside diameter (for example, on the insider diameter of hole 42) or on the outside diameter of ring 40. In one aspect of the invention, annular retaining structure 52 may comprise one or more annular recesses or one or more annular protections, for example, one substantially continuous annular recess or projection. For instance, as shown in FIGURE 6, annular retaining structure 52 may comprise an annular recess 53 adapted to engage one or more annular projections on cover 33. Annular recess 53 may have one or more internal corners or be radiused as shown in FIGURE 5. In one aspect, the engagement of annular retaining structure 52 with its complementary structure on cover 33 may be substantially fluid tight, that is, be so structured to minimize or prevent the passage of a fluid, for example, a liquid or a gas. In another aspect of the invention, for example, where fluid isolation is not paramount, the engagement between annular retaining structure 52 and its complementary structure may not be fluid tight. For example, in one aspect, annular retaining structure 52 may comprise a plurality of spaced recesses or projections, for instance, a plurality of spaced recesses or projections adapted to engage a plurality of complementary spaced recesses or projections in cover 33.

As shown in FIGURE 5, cover 33 is shown in larger scale to illustrate details of the invention. Cover 33 includes a thin membrane portion 54 and an annular or ring portion 56. According to one aspect of the invention, annular portion 56 provides means for mounting cover 33 to cap 32 and membrane portion 54 provides the protective barrier for hole 40 (and septum 35) in cap 32. Cover 33 also includes an outside diameter 60.

In a fashion similar to annular retaining structure 52 of cap 32, cover 33 includes an annular retaining structure 58, for example, a second annular retaining structure, adapted to engage an annular retaining structure, for example, a second annular retaining structure, in cap 32, for example, annular retaining structure 52 discussed above. In one aspect, the annular retaining structure 58 of cover 33 may be associated with annular or ring portion 56, for example, annular retaining structure 58 may be mounted on the inside diameter or the outside diameter of annular portion 58. In one aspect of the invention, annular retaining structure 58 may comprise one or more annular recesses or one or more annular projections, for example, one substantially continuous annular recess or projection. For instance, as shown in FIGURE 5, annular retaining structure 58 may comprise an annular projection 59 adapted to engage one or more annular recesses 53 in cap 32. In one aspect, as discussed above with respect to cap 32, the engagement of annular retaining structure 58 with its complementary structure on cap 32 may be substantially fluid tight or non-fluid tight. In another aspect of the invention, annular retaining structure 58 in cover 33 may comprise a plurality of spaced recesses or projections, for instance, a plurality of spaced recesses or projections adapted to engage a plurality of complementary spaced recesses or projections in cap 32. In one aspect, the first annular retaining structure 52 in cap 32 and second annular retaining structure 58 in cover 33 may comprise the only means of engagement between cap 32 and cover 33.

According to an aspect of the present invention, cover 33 is removably mounted to cap 32 and occludes opening 42 in ring 40 of cap 32. Moreover, cover 33 may include substantially no structure extending beyond the first or upper outside diameter 46 of skirt 34. For example, cover 33 may be so mounted to cap 32 that the outside diameter 60 of cover 33 is substantially no greater than the first outside diameter 46 of skirt 34. By providing cover 33 within the dimensions of cap 32, several advantages are provided, including limiting the modification of the molding tooling required to fabricate cap 32 and ensuring compatibility with existing cap and bottle handling equipment, for example, automated handling equipment, among other advantages. It is to be understood that, though in one aspect of the invention, outside diameter 60 of cover 30 may be substantially no greater than the first outside diameter 46 of skirt 34, practical considerations of manufacturing tolerances, material flexibility, and other material deviations, among other things, may create an outside diameter 60 somewhat greater than first outside diameter 46. Since such minor deviations may still effect one aspect of the desired invention, that is, compatibility with existing tooling and handling equipment, it will be understood by those of skill in the art that such deviations are within the scope of the present invention.

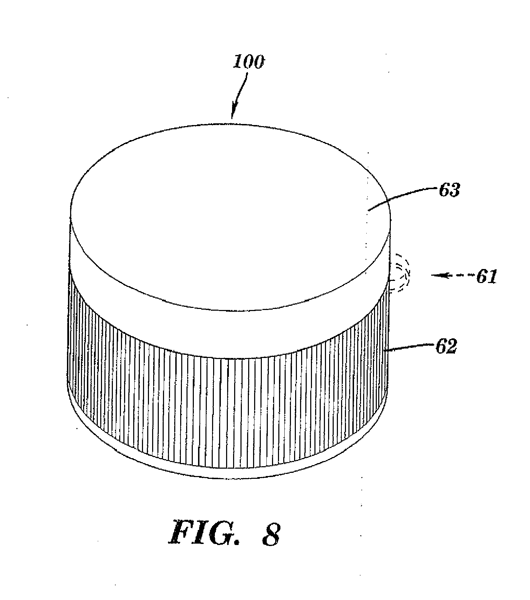

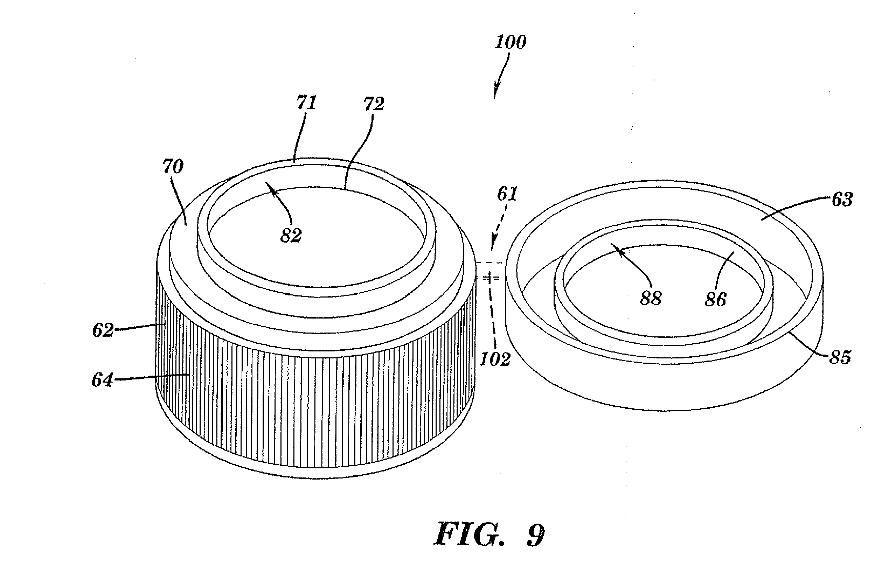

FIGURE 8 is perspective view of container top 100 according to another aspect of the invention. According to this aspect of the present invention, container top 100 includes a cap 62 and a cover 63. In one aspect, cover 63 may engage cap 62 in a fashion similar to the way cover 33 engages cap 32 above, that is, by means of complementary annular retaining structures 52 and 58. However, contrary to the aspect shown in FIGURES 3-7, container top 100 may include a hinge assembly 61 (shown in phantom) adapted to retain cover 63 when cover 63 is disengaged from cap 62. FIGURE 9 is a perspective view of the container top 100 shown in FIGURE 8 with the cover 63 in the opened position, for example, as retained by hinge assembly 61.

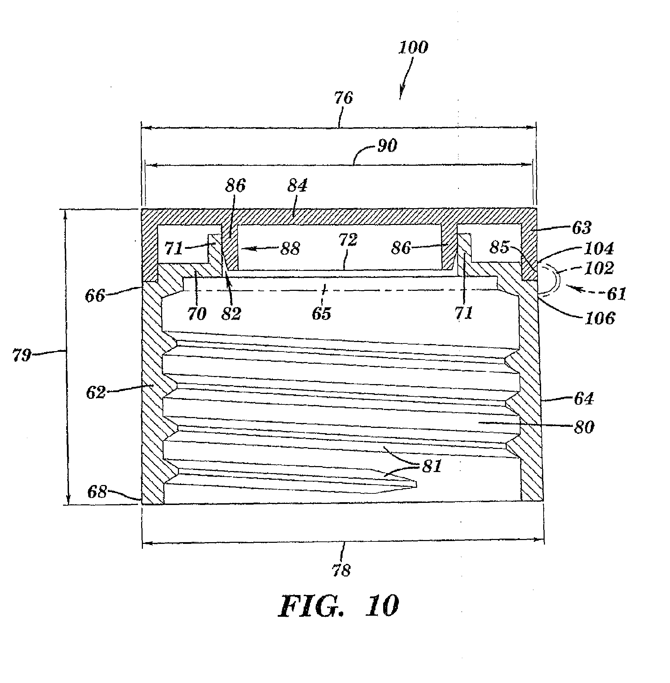

FIGURE 10 is a cross sectional view of container top 100 shown in FIGURES 8 and 9. In one aspect of the invention, cap 62 may include a septum 65 (shown in phantom in FIGURE 10), for example, an elastomeric septum similar to septa 22 and 35 discussed above.

As shown most clearly in FIGURE 10, cap 62 includes a cylindrical skirt 64 having a top or first end 66 and a bottom or second end 68, and a ring 70 mounted to the top 66. Ring 70 includes an opening or hole 72 which exposes septum 65 (again, shown in phantom in FIGURE 10) mounted within cap 62, for example, by means of the methods disclosed in U.S. patents 5,647,939 and 6,234,335. According to an aspect of the invention, skirt 34 includes a first outside diameter 76 at the top 66 of skirt 64, a second outside diameter 78 at the bottom 68 of skirt 64, a height 79, and an inside surface 80 adapted to engage a container (not shown), for example, a bottle similar to bottle 14 shown in FIGURES 1 and 2. The inside diameter 80 may include one or more helical threads 81 adapted to engage a threaded bottleneck. In one aspect, first outside diameter 76 may be substantially equal to second outside diameter 78; however, first outside diameter 76 may be smaller than second outsider diameter 78, for instance, to facilitate removal of cap 62 from a mold when cap 62 is fabricated by molding. Outside diameter 76 may also be greater than outside diameter 78.

As also shown in FIGURE 10, cap 62 may include an annular retaining structure 82, for example, a first annular retaining structure, adapted to engage an annular retaining structure, for example, a second annular retaining structure, in cover 63, for example, annular retaining structure 88 on cover 63. In one aspect, the annular retaining structure 82 of cap 62 may be associated with ring 70, for example, annular retaining structure 82 may be mounted on the inside diameter (for example, on the inside diameter of hole 72) or the outside diameter of ring 70, for example, a ring 71 mounted to or projecting from ring 70. In one aspect of the invention, annular retaining structure 82 may comprise one or more annular recesses or one or more annular protections, for example, one substantially continuous annular recess or projection or one or more discontinuous annular recesses or projections (as discussed above with respect to container cover 10) mounted in or on ring 71. Similarly, in one aspect of the invention, annular retaining structure 88 on cover 63 may comprise one or more annular recesses or one or more annular protections, for example, one substantially continuous annular recess or projection or one or more discontinuous annular recesses or projections (as discussed above with respect to container cover 10) mounted in or on ring 86 (described below). In one aspect, the engagement of annular retaining structure 82 with its complementary retaining structure 88 on cover 63 may be substantially fluid tight or non-fluid tight, depending upon the medium container top 100 is used to retain and the application of container top 100.

As shown in FIGURE 10, cover 63 may include a thin membrane portion 84 and an outer annular or ring portion 85 and an inner annular ring portion 86. According to one aspect of the invention, inner annular portion 86 may provide means for removably engaging cover 63 to cap 62 and membrane portion 84 may provide a protective barrier for hole 72 (and septum 65) in cap 62. Cover 63 outer annular ring 85 also includes an outside diameter 90.

As also shown in FIGURE 10, annular retaining structure 88 on cover 63 is adapted to removably engage annular retaining structure 82 on cap 62. In the aspect shown in FIGURE 10, though the retaining structures may comprise one or more recesses or projections as discussed above, annular retaining structures 82 on cap 62 and annular retaining structure 88 on cover 63 may comprise a friction fit between annular rings 71 and 86. For example, rings 71 and 86 may be sized whereby sufficient friction is provided between their respective contacting surfaces to provide sufficient engagement to minimize or prevent disengagement. In one aspect, the annular retaining structure 82 in cap 64 and annular retaining structure 88 in cover 63 may comprise the only means of engagement between cap 62 and cover 63, for example, friction between rings 71 and 86 may be the only means of engagement between cap 62 and cover 63.

Similar to the aspect of the invention illustrated in and described with respect to FIGURES 3 to 7, according to an aspect of the present invention, cover 63 is removably mounted to cap 62 and occludes opening 72 in ring 70 of cap 62. Moreover, in one aspect, cover 63 may include substantially no structure extending beyond the first or upper outside diameter 76 of skirt 64. For example, cover 63 may be so mounted to cap 62 that the outside diameter 90 of cover 63 is substantially no greater than the first outside diameter 76 of skirt 64. By providing cover 63 within the dimensions of cap 62, several advantages are provided, including limiting the modification of the molding tooling required to fabricate cap 62 and ensuring compatibility with existing cap and bottle handling equipment, for example, automated handling equipment, among other advantages.

As also shown in FIGURE 10, container top 100 may include some form of hinge assembly 61 (shown in phantom) adapted to retain cover 63 on cap 62 when cover 63 is dislodged from engagement with cap 63. For example, as shown in FIGURE 10, in one aspect, hinge assembly 61 may comprise a strap 102 having a first end 104 mounted to cover 63 and a second end 106 mounted to cap 62. In this aspect of the invention, hinge assembly 61 may retain cover 63 on cap 62 when cover 63 is removed from or disengaged from cap 62 to expose opening 72 in cap 62. In one aspect, cover 63, cap 62, and strap 102 may be fabricated as one integral component, for example, by molding. Strap 102 may have a length of between about 0.25 inches and about 0.50 inches; a width of between about 0.10 inches and about 0.25 inches; and a thickness of between about 0.005 inches and about 0.050 inches.

Container covers 10 and 100 may be fabricated from any conventional metallic or non-metallic material, but is typically fabricated from plastic. For example, container covers 10 and 100 may be fabricated from one or more of the following plastics: a polyamide (PA), for example, nylon; a polyamide-imide; a polyethylene (PE); a polypropylene (PP); a polyester (PE); a polytetraflouroethylene (PTFE); an acrylonitrile butadiene styrene (ABS); a polycarbonate (PC); or a vinyl, such as, polyvinylchloride (PVC), among other plastics.

The outside diameter 60 of cover 33, the outside diameters 46 and 48 of cap 32, the outside diameter 76 of cover 63, and the outside diameter 90 of cap 62 may vary from about 0.25 inches to about 12 inches, but are typically between about 0.5 inches and about 3 inches, for example, about 1 inch. The thickness of membrane portion 54 of cover 33 and the thickness of membrane portion 84 of cover 63 may vary from about 0.01 inches to about 1.0 inch, but is typically between about 0.010 inches and about 0.020 inches, for example, about 0.015 inches. The height 49 of cap 32 and the height 79 of cap 62 may vary from about 0.25 inches to about 6 inches, but is typically between about 0.40 inches and about 0.75 inches, for example, about 0.60 inches.

According to aspects of the invention, container covers 10 and 100 provide improvements over the container covers according to the prior art, in particular, when container covers 10 and 100 include flexible septa. Aspects of these inventions minimize or eliminate the need to handle and wash separate dust covers while imposing little or no impact upon the manufacture and handling of such container covers. These and other advantages of aspects of the invention will be apparent to those of skill in the art.

While several aspects of the present invention have been described and depicted herein, alternative aspects may be effected by those skilled in the art to accomplish the same objectives. Accordingly, it is intended by the appended claims to cover all such alternative aspects as fall within the true spirit and scope of the invention.

## Claims

1. A container top comprising:
a cap (32) comprising:
a cylindrical skirt (34) having a top (36), a bottom (38), a first outside diameter (46) at the top (36) of the skirt (34), a second outside diameter (48) at the bottom (38) of the skirt (34), and an inside surface (50) adapted to engage a container; and
a ring (40) mounted to the top (36) of the cylindrical skirt (34), the ring (40) having an opening (42); and
a cover (33) removably mounted to the cap (32) and occluding the opening (42) in the ring (40), the cover (33) having substantially no structure extending beyond the first outside diameter (46) of the skirt (34).

2. The container top as recited in claim 1, wherein the container top further comprises a septum (35) positioned in the cap (32) to occlude the opening (42) in the ring (40).

3. The container top as recited in claim 1 or claim 2, wherein the ring (40) comprises a first annular retaining structure (52) and the cover (33) comprises a second annular retaining structure (58) adapted to engage the first annular retaining structure (52) to retain the cover (33) on the cap (32).

4. The container top as recited in claim 3, wherein the first annular retaining structure (52) on the ring (40) comprises an annular recess (53) and the second annular retaining structure (58) on the cover (33) comprises an annular projection (59) adapted to engage the annular recess (53).

5. The container top as recited in claim 4, wherein the ring (40) comprises an inside diameter and an outside diameter, and wherein the annular recess (53) on the ring (40) is positioned on one of the insider diameter and the outside diameter of the ring (40).

6. The container top as recited in claim 5, wherein the annular recess (53) is positioned on the outside diameter of the ring (40).

7. The container top as recited in any one of claims 3 to 5, wherein the first annular retaining structure (52) on the ring (40) comprises an annular projection and the second annular retaining structure (58) on the cover (33) comprises an annular recess adapted to engage the annular projection on the ring (40).

8. The container top as recited in claim 7, wherein the ring (40) comprises an inside diameter and an outside diameter, and wherein the annular projection on the ring (40) is positioned on one of the insider diameter and the outside diameter of the ring (40).

9. The container top as recited in claim 8, wherein the annular projection is positioned on the outside diameter of the ring (40).

10. The container top as recited in any one of claims 1 to 9, wherein the second outside diameter (48) of the skirt (34) is greater than the first outside diameter (46) of the skirt (34).

11. The container top as recited in any one of claims 1 to 10, wherein the first annular retaining structure (52) and the second annular retaining structure (58) comprise the only means of engagement between the cap (32) and the cover (33).

12. A method of protecting a cap septum comprising:
providing a cap (32) comprising:
a cylindrical skirt (34) having top (36), a bottom (38), a first outside diameter (46) at the top (36) of the skirt (34), and a second outside diameter (48) at the bottom (38) of the skirt (34); and
a ring (40) mounted to the top (36) of the cylindrical skirt (34), the ring (40) having an opening (42);
inserting a septum (35) into the cap (32) wherein the septum (35) occludes the opening (42);
mounting the cap (32) with the septum (35) on a container wherein at least some of the septum (35) is exposed through the opening (42) in the ring (40); and
mounting a removable cover (33) on the cap (32) to protect the septum (35) while providing no structure extending beyond the first outside diameter (46) of the skirt (34).

13. The method as recited in claim 12, wherein the cap (32) further comprises a first annular retaining structure (52) and the cover (33) comprises a second annular retaining structure (58), and wherein mounting the removable cover (33) on the cap (32) comprise engaging the first annular retaining structure (52) with the second annular retaining structure (58).

14. The method as recited in claim 13, wherein the first annular retaining structure (52) on the cap (32) comprises an annular recess (53) and the second annular retaining structure (58) on the cover (33) comprises an annular projection (59), and wherein engaging the first annular retaining structure (52) with the second annular retaining structure (58) comprises engaging the annular recess (53) and the annular projection (59).

15. The method as recited in claim 13, wherein the first annular retaining structure (52) on the cap (32) comprises an annular projection and the second annular retaining structure (58) on the cover (33) comprises an annular recess, and wherein engaging the first annular retaining structure (52) with the second annular retaining structure (58) comprises engaging the annular projection and annular recess.
